# EUROPEAN PATENT SPECIFICATION

(11) **EP 2 526 977 B1**
(45) Date of publication and mention of the grant of the patent: **26.02.2014**
(21) Application number: 12153261.8
(22) Date of filing: 31.01.2012
(51) Int. Cl.: A61L 27/18, A61L 27/30, A61F 2/24

(54) **A PEEK MEDICAL IMPLANT AND A METHOD OF FORMATION OF SURFACE LAYERS ON MEDICAL IMPLANTS**
MEDIZINISCHES PEEK IMPLANTAT MIT VERSCHIEDENEN BESCHICHTUNGEN UND VERFAHREN ZU SEINER HERSTELLUNG
IMPLANT MÉDICAL EN PEEK REVETU ET SA MÉTHODE DE FABRICATION.

(30) Priority: 28.02.2011 PL 39405411
(43) Date of publication of application: 28.11.2012
(73) Proprietor: Fundacja Rozwoju Kardiochirurgii Im. Prof. Zbigniewa Religi, 41-800 Zabrze (PL)
(72) Inventor: Wierzchon, Tadeusz, 02-642 Warszawa (PL); Dalczynska-Jonas, Stanislawa, 31-228 Kraków (PL); Tkacz-Smiech, Katarzyna, 30-198 Kraków (PL); Borowski, Tomasz, 05-820 Piastów (PL); Gonsior, Malgorzata, 41-806 Zabrze (PL); Kustosz, Roman, 41-800 Zabrze (PL)
(74) Representative: Malcherek, Piotr

(56) References cited:
- GB-A- 2 452 518
- DATABASE WPI Week 200008 Thomson Scientific, London, GB; AN 2000-094817 XP002685397, & RU 2 115 388 C1 (GRANIT RES INST) 20 July 1998 (1998-07-20)
- Gonsior ET AL: "Thrombogenicity of Ti(N,C,O) diffusive coating layers developed on Titanium alloy as the blood contact surface", European Cells and Materials vol 19, supplement 1, 1 January 2010 (2010-01-01), pages 32-32, XP55041040, Retrieved from the Internet: URL:http://www.ecmjournal.org/journal/supp lements/vol019supp01/pdf/v019supp01a032.pd f [retrieved on 2012-10-15]

## Description

The invention relates to a medical implant, in particular an element of a mechanical heart valve and a method for formation of surface layers on medical implants, in particular on elements of mechanical heart valves made from polyetheretherketone.

The main element of a mechanical heart valve is a movable disc which opens in the blood stream which is also a closing mechanism of a heart valve. Discs of this type and other medical implants are made of various materials including polymers, for instance, of polyetheretherketone with a trade name PEEK, polyoxymethylene known as Delrin, polytetrafluoroethylene known as Teflon. Moreover, pyrolytic carbon is also known to be used as an external layer formed on polycrystalline graphite or titanium and its alloys with a layer of nanocrystalline diamond - NCD, formed according to the patent description PL 186562. Coatings of amorphous hydrogenated carbon - a-C:H, also nitrogen a-C:N:H, are also used as well as layers of amorphous hydrogenated silicon carbide - a-SiC(H), used, for instance, on the surface of stents.

The applied materials should be highly resistant, ensure minimum resistance to blood flow, they should be resistant to friction wear, mechanically resistant, resistant to degradation and sterilization as well as biocompatible which means they should have the lowest possible impact on the morphological elements of the blood and endothelial cells surrounding the valve, they should carry minimum risk of thrombogenesis and eliminate adsorption of blood elements. Therefore, the materials selected for making a mechanical valve, as well as the structure thereof, are the essential prerequisite for its song life.

The invention relates to a medical implant, in particular an element of a mechanical heart valve, made of polyetheretherketone. The essence of the invention is that the implant has a gradient surface layer comprising the following coatings from the polyetheretherketone base:
- an amorphous nitrogen doped hydrogenated carbon coaling - a-C:N:H;
- an amorphous or amorphous-nanocrystal line nitrogen and silicon doped hydrogenated carbon coating - a-C:N:H(Si) or C:N:H(Si);
- and amorphous or nanocrystalline or amourphous-nanocrystalline silicon carbonitride coaling - a-SiCN(H) or SiCN(H);
wherein the gradient surface layer comprising the above coatings is formed as a result of argon ion etching of the implant in the atmosphere containing precursors of carbon, nitrogen and silicon and argon as carrier gas in the temperature within the range between 22 °C and 100°C at pressure in the working chamber within the range between 0. and 2 millibar with the use of chemical vapour deposition from the gas phase with the use of chemical reactions with the use of curreiit-generated plasma or microwave-generated plasma.

The gradient layer is a layer in which the concentration of the substance forming the layer in relation to the base substance increases continually in the direction where the gradient points. According to the invention the concentration of the substance forming the surface layer in relation to the base substance decreases continually to 0 in the direction from the surface towards the base.

It is preferable that the overall thickness of the gradient surface layer does not exceed 1000 n-m. The content ratio of the number of atoms of carbon to nitrogen can range from 9 to 4 throughout the layer and the concentration of hydrogen in the layer is no more than 30% of the number of atoms, The concentration of silicon in the direction perpendicular to the polyetheretherketone base to the external surface of the layer increases from 0 to maximum 25% of the number of atoms.

In a preferred embodiment the thickness of the coating a-C-N:H is no more than 40% of the thickness of the gradient surface layer, preferably 37.5%, the thickness of the a-C:N-H(Si) or C:N:H(Si) coating does not exceed 55% of the thickness of the gradient surface layer, preferably 50% and the thickness of the a-SiCN(H) or SiCN(H) coating does not exceed 20% of the gradient surface layer, preferably 17.5%.

Moreover, the gradient of the increasing concentration of silicon is directed towards the surface of the layer and the gradient of concentration of nitrogen and carbon is directed towards the polyetheretherketone base.

Another invention relates to the method for formation of surface layers on medical implants made from polyetheretherketone, in particular on elements of mechanical heart valves. The essence of the invention is that on a prepared implant a gradient surface layer is formed, which surface layer comprises the following coatings starting from the polyetheretherketone base:
- an amorphous hydrogenated nitrogen doped carbon coaling - a-C:N:H;
- an amorphous or amorphous-nanocrystalline hydrogenated nitrogen and silicon doped carbon coaling - a-C:N:H(Si) or C:N:H(Si);
- and an amorphous or nanocrystalline or amorphous-nanocrystalline silicon carbonitride coaling - a-SiCN(H) or SiCN(H)_{;}
by argon ion etching of the implant in the atmosphere containing precursors of carbon, nitrogen and silicon as well as argon as carrier gas in the temperature within the range between 22°C and 100°C, at pressure in a working chamber within the range between 0.1 and 2 millibar with the use of chemical vapour deposition from gas phase with the use of chemical processes with the use of current- generated plasma or microwave-generated plasma.

In a preferred embodiment the etching should be carried out in the temperature within the range between 30°C and 60°C, most preferably 40°C.

It is also recommendable that the etching is carried out at pressure within the range between 0.2 millibar and 0.4 millibar.

The surface layer of the proposed structure adheres well to the base. Gradient silicon carbonitride - SiCₓN_{y}(H) coatings have better tribological and biological properties in contact with blood compared to other known layers. The layer whose thickness is up to 1000 nm can be formed on elaborately shaped elements. The method according to the invention to form said gradient surface layers with the external hydrogenated silicon carbonitride coating containing: carbon, nitrogen, silicon and hydrogen of different concentration formed by PACVD method which is a method of chemical vapour deposition from gas phase with the radio-frequency or microwave current activation of the reactive atmosphere. The formed layers adhere well to a polyetheretherketone base and the friction coefficient thereof is reduced nearly threefold in relation to non-modified polymer. The so formed layer also reduces adhesion of platelets and aggregates of blood platelets.

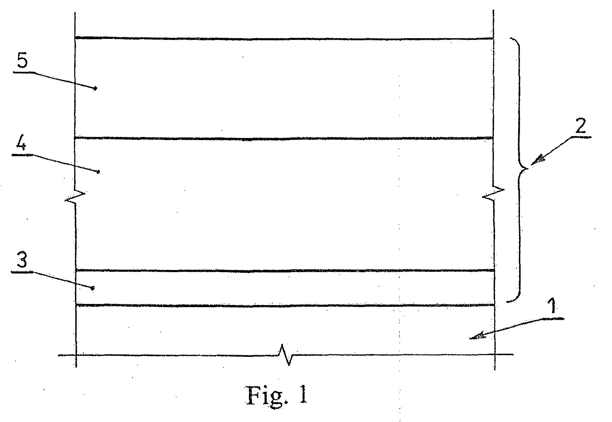

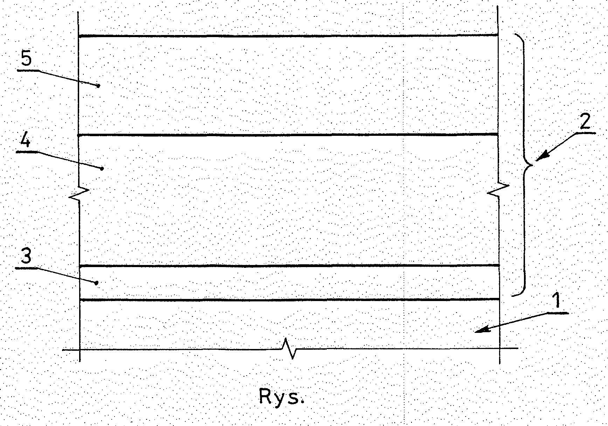

A medical implant and a method for formation of surface layers on medical implants are presented in more detail in the following embodiments and in the enclosed drawing which shows a schematic cross section of a fragment of the mechanical heart valve with schematic coatings of the gradient surface layer.

Disc 1 of the mechanical heart valve is made from polyetheretherketone and has an external gradient layer 2 comprising the following coatings from the polyetheretherketone base: an amorphous hydrogenated nitrogen doped carbon coating 3 - a-C:N:H approximately 300 nm thick, an amorphous hydrogenated silicon doped carbon coaling 4 - a-C:N-H(Si) approximately 400 nm thick and an external amorphous silicon carbonitride coating 5 - a-SiCN(I-1) approximately 100 nm thick. The ratio of the number of carbon atoms to the number of nitrogen atoms in the gradient layer is approximately 6, and the hydrogen content is 25% of the number of atoms. Moreover, the concentration of silicon in the direction perpendicular towards the polyetheretherketone base increases from the base towards the surface of the layer frog 0 to approx. 23.5 % in the number of atoms, Furthermore, the nitrogen and carbon concentration gradient is directed towards the polyetheretherketone base.

In alternative embodiments the amorphous hydrogenated nitrogen and silicon doped carbon coating 4 - a-C:N:H(Si) may be replaced with an amorphous-nanocrystalline hydrogenated nitrogen and silicon doped carbon coaling - C:N:H(Si). Additionally, the amorphous nanocrystalline silicon carbonitride coating 5 - a-SiCN(H) may be replaced with a nanocrystlline or amorphous-nanocrystalline silicon carbonitride coaling - SiCN(H).

In further of the invention the thickness of the layer 2 may be up to 1 000nm, wherein the thickness of the coating 3 a-C:N:H may be up to 40% of the thickness of the layer 2, the thickness of the coating 4 a-C:N:H(Si) or C:N:H(Si) may be up to 55% of the thickness of the layer 2, and the thickness of the coating 5 a-SiCN(H) or SiCN(.lX) may be up to 20% of the thickness of the layer 2.

Additionally, in further embodiments of the invention the ratio of the number of atoms of carbon to the number of atoms of nitrogen in coatings of the layer 2 may range between 9 and 4, and the concentration of hydrogen may be up to 30 % of the number of atoms. The concentration of silicon in direction perpendicular towards the polyetheretherketone base towards the surface of the external layer may rise to the maximum of 25% of the number of atoms.

The gradient surface layer of a medical implant made from polyetheretherketone, for example a disc of a mechanical heart valve, another element of a heart valve or another medical implant made from polyetheretherketone is formed in the following way. First, an implant is mechanically machined and degreased by ultrasonic cleaning in acetone. Then the so prepared implant undergoes argon Ar⁺ ion etching in an RFCVD chamber which is a chamber for chemical vapour deposition from gas phase by radio-frequency generated plasma, whose work parameters are for example: frequency 13.56 MHz, and power 400 W. After that the implant undergoes a process of chemical vapour deposition from the gas phase with the use of chemical reactions in reactive environment containing precursors of carbon, nitrogen and silicon, respectively: CH₄, N₂, SiH₄ in the presence of argon as carrier gas activated by high frequency current - 13.56 MHz, 400 W, at pressure within the range between 0.2 and 0.4 millibar, at the temperature of 40°C during 30 minutes.

Alternatively, instead of using an RFCVD chamber an MWCVD chamber may be used which means that an implant may undergo etching in a chamber for chemical vapour deposition from the gas phase with the use of microwave generated plasma, whose work parameters are for example: frequency 2.45 GHz, power 2 kW.

In other embodiments of the method an implant undergoes the process of chemical vapour deposition from the gas phase at the temperature within the range between 22°C and 100°C, and pressure in the working chamber within the range between 0.1 and 2 milibar.

The scope of protection includes implants whose selected surfaces comprise a gradient surface layer in variants described above, in particular at least surfaces designed to be in contact with blood.

## Claims

1. A medical implant, in particular an element of a mechanical heart valve, made from polyetheretherketone, **characterised in that** it has a gradient surface layer ( 2 ), comprising the following coatings from the base of the implant:
- an amorphous hydrogenated nitrogen doped carbon coating ( 3 ) - a-C:N:H;
- an amorphous or amorphous-nanocrystalline hydrogenated nitrogen and silicon doped carbon coating ( 4 ) - a-C:N:H(Si) or C:N:H(Si);
- an amorphous or nanocrystalline or amorphous-nanocrystalline silicon carbonitride coating ( 5 ) - a-SiCN(H) or SiCN(H);
wherein the gradient surface layer comprising the above coatings is formed as a result of argon ion etching of the implant in the atmosphere containing precursors of carbon, nitrogen, silicon and argon as carrier gas in the temperature within the range between 22°C and 100°C at pressure in a working chamber within the range between 0.1 and 2 millibar with the use of chemical vapour deposition from gas phase with the use of chemical reactions with the use of current-generated plasma or microwave-generated plasma

2. The implant according to claim 1 **characterised in that** the overall thickness of the gradient surface layer ( 2 ) is maximum 1000 nm, and the ratio of the number of carbon atoms to the number of nitrogen atoms is within the range between 9 and 4, and the concentration of hydrogen in the layer is maximum 30 % of the number of atoms, and the concentration of silicon in the direction perpendicular to the polyetheretherketone base towards the surface of the layer increases within the range from 0 to maximum 25% of the number of atoms.

3. The implant according to claims 1 or 2, **characterised in that** the thickness of the coating ( 3 ) a-C:N:H does not exceed 40% of the thickness of the gradient surface layer ( 2 ), preferably 37.5 %, the thickness of the coating ( 4 ) a-C:N:H(Si) or C:N:H(Si) does not exceed 55% of the thickness of the gradient surface layer ( 2 ), preferably 50 %, and the thickness of the coating ( 5 ) a-SiCN(H) or SiCN(H) does not exceed 20% of the thickness of the gradient surface layer ( 2 ), preferably 17.5 %.

4. The implant according to one of the claims 1-3, **characterised in that** the gradient of the increasing silicon concentration is directed towards the surface of the layer, whereas the gradient of nitrogen and carbon concentration is directed towards the polyetheretherketone base.

5. A method for formation surface layers on medical implants made from polyetheretherketone, in particular on elements of mechanical heart valves, **characterised in that** on the prepared implant a gradient surface layer is formed and said gradient layer comprises the following coatings from the polyetheretherketone base of the implant:
- an amorphous hydrogenated nitrogen doped carbon coating - a-C:N:H;
- an amorphous or amorphous-nanocrystalline hydrogenated nitrogen and silicon doped carbon coating - a-C:N:H(Si) or C:N:H(Si);
- an amorphous or nanocrystalline or amorphous-nanocrystalline silicon carbonitride coating - a-SiCN(H) or SiCN(H)
by argon Ar⁺ ion etching of the implant in an atmosphere containing precursors of carbon, nitrogen, silicon and argon as carrier gas at the temperature within the range between 22°C and 100°C, at pressure in a working chamber within the range between 0.1 and 2 milibar with the use of chemical vapour deposition from gas phase with the use of chemical reactions with the use of current-generated plasma or microwave-generated plasma.

6. The method according to claim 5 **characterised in that** the etching is carried out at the temperature within the range between 30°C and 60°C, most preferably 40°C.

7. The method according to claims 5 or 6, **characterised in that** the etching is carried out at pressure within the range between 0.2 milibar and 0.4 milibar.

## Patentansprüche

1. Medizinisches Implantat, insbesondere das Element der mechanischen Herzklappe, erstellt aus Polyetheretherketon, **gekennzeichnet dadurch, dass** es die Gradienten-Oberflächenschicht /2/ aufweist, die von der Implantatsbasis her folgende Schichten umfasst:
- amorphe, hydrierte, mittels Stickstoff - a-C:N:H angereichte Kohleschicht /3/;
- amorphe oder amorph-nanokristallinische, hydrierte, mittels Stickstoff und Silizium - a-C:N:H(Si) oder C:N:H(Si) angereichte Kohleschicht /4/;
- amorphe oder nanokristallinische oder amorph-nanokristallinische Silizium Carbonitridschicht /5/ - a-SiCN(H) oder SiCN(H),
wobei die Gradienten-Oberflächenschicht, die die obigen Schichten umfasst, in Ätzbehandlung des Implantats mittels Argon Ion Ar+ in der Atmosphäre, die Vorläufer von Kohle, Stickstoff, Silizium und Argon als Traggas beinhaltet, in der Temperatur aus dem Bereich von 22°C bis 100°C, unter Druck in der Betriebskammer aus dem Bereich von 0,1 bis 0,2 Millibar, unter Verwendung des chemischen Ahlagerungsprozesses aus der Gasphase mit Teilnahme von chemischen Reaktionen bei Anwendung von mittels Strom bzw. Mikrowellen generierten Plasma hergestellt ist.

2. Medizinisches Implantat nach Anspruch 1 **dadurch gekennzeichnet, dass** die Gesamtdicke der Gradienten-Oberflächenschicht /2/ bis 1000 nm beträgt, und das Mengenverhältnis der Kohleatome zu Stickstoffatome im Bereich von 9 bis 4 enthalten ist, und die Wasserstoffkonzentration in der Schicht bis 30% der Atommenge beträgt, und die Siliziumkonzentration dagegen senkrecht gerichtet von der Implantatsbasis aus Polyetheretherketon zur Oberfläche der äußeren Schicht hin im Bereich von 0 bis max. 25% der Atommenge zunimmt.

3. Medizinisches Implantat nach Anspruch 1 oder 2 **dadurch gekennzeichnet, dass** die Dicke der Schicht /3/ a-C:N:H bis 40% der Dicke der Gradienten-Oberflächenschicht /2/, vorzüglich 37,5%, beträgt, die Dicke der Schicht /4/ a-C:N:H(Si) oder C:N:H(Si) bis 55% der Dicke der Gradienten-Oberflächenschicht /2/, vorzüglich 50%, beträgt, und die Dicke der Schicht /5/ a-SiCN(H) oder SiCN(H) 20% der Dicke der Gradienten-Oberflächenschicht /2/, vorzüglich 17,5%, beträgt.

4. Medizinisches Implantat nach einem der obigen Ansprüche 1-3 **dadurch gekennzeichnet, dass** der Gradient der wachsenden Siliziumkonzentration zur Schichtoberfläche gerichtet ist, aber der Gradient der Stickstoff- und Kohlekonzentration zu der aus Polyetheretherketon gefertigten Implantatsbasis gerichtet ist.

5. Verfahren für Fertigung von Oberflächenschichten an den aus Polyetheretherketon hergestellten medizinischen Implantaten, insbesondere an Elementen der mechanischen Herzklappen, **dadurch gekennzeichnet, dass** auf dem vorbereiteten Implantat die Gradienten-Oberflächenschicht, die von der Implantatzbasis aus Polyetheretherketon her folgende Schichten:
- amorphe, hydrierte, mittels Stickstoff - a-C:N:H angereichte Kohteschicht /3/;
- amorphe oder amorph-nanokristallinische, hydrierte, mittels Stickstoff und Silizium - a-C:N:H(Si) oder C:N:H(Si) angereichte Kohleschicht /4/;
- und amorphe oder nanokristallinische oder amorphnanokristallinische Silizium Carbonitridschicht /5/ - a-SiCN(H) oder SiCN(H)
umfasst und in Ätzbehandlung des Implantats mittels Argon Ion Ar+ in der Atmosphäre, die Vorläufer von Kohle, Stickstoff, Silizium und Argon als Traggas beinhaltet, in der Temperatur aus dem Bereich von 22°C bis 100°C, unter Druck in der Betriebskammer aus dem Bereich von 0,1 bis 0,2 Millibar, unter Verwendung des chemischen Ablagerungsprozesses aus der Gasphase mit Teilnahme von chemischen Reaktionen bei Anwendung von mittels Strom bzw. Mikrowellen generierten Plasma hergestellt ist.

6. Verfahren nach Anspruch 5 **dadurch gekennzeichnet, dass** die Ätzung in der Temperatur aus dem Bereich von 30°C bis 60°C, am günstigsten 40°C, durchgeführt ist.

7. Verfahren nach Anspruch 5 oder 6 **dadurch gekennzeichnet, dass** die Ätzung unter Druck aus dem Bereich von 0,2 Millibar bis 0,4 Millibar durchgeführt ist.

## Revendications

1. Un implant médical, en particulier un élément de la valve cardiaque mécanique, fait en polyétheréthercétone, **caractérisé en ce qu'**il a une couche superficielle à gradient (2), comprenant, dans le sens à partir du substrat :
- un revêtement de carbone amorphe hydrogéné (3) dopé de l'azote - a-C:N:H;
- un revêtement de carbone amorphe ou amorphe-nanocristallin hydrogéné (4) dopé de l'azote et du silicium - a-C:N:H(Si) ou C:N:H(Si);
- un revêtement de carbonitrure de silicium amorphe ou nanocristallin, ou amorphe-nanocristallin (5) - a-SiCN(H) ou SiCN(H);
où la couche superficielle à gradient, comprenant les revêtements ci-dessus est formée par attaque de l'implant avec des ions d'argon Ar⁺ dans l'atmosphère contenant des précurseurs du carbone, du silicium et de l'azote, ainsi que de l'argon en tant que gaz porteur à une température comprise dans la plage de 22 °C à 100 °C, à une pression dans la chambre de travail de 0,1 à 2 mbar, en utilisant le processus de sédimentation chimique en phase vapeur, impliquant des réactions chimiques avec utilisation de plasma généré par des courants ou avec utilisation de plasma généré par des micro-ondes.

2. L'implant selon la revendication 1, **caractérisé en ce que** l'épaisseur totale de la couche superficielle à gradient (2) s'élève à 1000 nm, et le rapport du nombre d'atomes de carbone à celui d'azote est dans la plage de 9 à 4, et la concentration en hydrogène dans la couche va jusqu'à 30% du nombre d'atomes, tandis que la concentration en silicium dans le sens perpendiculaire à partir du substrat en polyétheréthercétone vers la surface de la couche extérieure augmente de 0 à un maximum de 25 % du nombre d'atomes.

3. L'implant selon la revendication 1 ou 2, **caractérisé en ce que** l'épaisseur du revêtement (3) a-C:N:H s'élève jusqu'à 40 % de l'épaisseur de la couche superficielle à gradient (2), de préférence 37,5 %, l'épaisseur du revêtement (4) a-C:N:H(Si) ou C:N:H(Si) s'élève jusqu'à 55 % de l'épaisseur de la couche superficielle à gradient (2), de préférence 50 %, et l'épaisseur du revêtement (5) a-SiCN(H) ou SiCN(H) s'élève jusqu'à 20% de l'épaisseur de la couche superficielle à gradient (2), de préférence 17,5 %.

4. L'implant selon l'une des revendications précédentes 1 à 3, **caractérisé en ce que** le gradient de concentration croissante en silicium est orienté vers la surface de la couche, et le gradient de concentration en azote et en charbon est orienté vers le substrat fait en polyétheréthercétone.

5. Un procédé de formation des couches superficielles sur les implants médicaux faits en polyétheréthercétone, en particulier des éléments de valves cardiaques mécaniques, **caractérisé en ce que** sur l'implant préparé est formée une couche superficielle à gradient, qui couvre successivement dans le sens à partir du substrat en polyétheréthercétone :
- un revêtement de carbone amorphe hydrogéné (3) dopé de l'azote - a-C:N:H ;
- un revêtement de carbone amorphe ou amorphenanocristallin hydrogéné (4) dopé de l'azote et du silicium - a-C:N:H(Si) ou C:N:H(Si);
- et un revêtement de carbonitrure de silicium amorphe ou nanocristallin, ou amorphe-nanocristallin (5) - a-SiCN(H) ou SiCN(H);
par attaque de l'implant avec des ions d'argon Ar⁺ dans l'atmosphère contenant des précurseurs du carbone, du silicium et de l'azote, ainsi que de l'argon en tant que gaz porteur à une température comprise dans la plage de 22 °C à 100 °C, à une pression dans la chambre de travail de 0,1 à 2 mbar, en utilisant le processus de sédimentation chimique en phase vapeur, impliquant des réactions chimiques avec utilisation de plasma généré par des courants ou avec utilisation de plasma généré par des micro-ondes.

6. Le procédé selon la revendication 5, **caractérisé en ce que** l'attaque ionique est effectuée à une plage de température de 30 °C à 60 °C, de préférence 40 °C.

7. Le procédé selon les revendications 5 ou 6, **caractérisé en ce que** l'attaque ionique est effectuée à une pression allant de 0,2 mbar à 0,4 mbar.
